# EUROPEAN PATENT SPECIFICATION

(11) **EP 3 349 012 B1**
(45) Date of publication and mention of the grant of the patent: **05.05.2021**
(21) Application number: 17183618.2
(22) Date of filing: 27.07.2017
(51) Int. Cl.: G01N 33/68

(54) **USE OF A COMPOSITION FOR DIAGNOSING BEHAVIOURAL ADDICTION AND METHOD OF DETECTING SPHINGOSINE 1-PHOSPHATE RECEPTOR 1 FOR DIAGNOSIS OF BEHAVIOURAL ADDICTION**
VERWENDUNG EINER ZUSAMMENSETZUNG ZUR DIAGNOSE VON VERHALTENSBEZOGENER ABHÄNGIGKEIT UND VERFAHREN ZUR DETEKTION VON SPHINGOSIN-1-PHOSPHAT-REZEPTOR 1 ZUR DIAGNOSE VON VERHALTENSBEZOGENER ABHÄNGIGKEIT
UTILISATION D'UNE COMPOSITION PERMETTANT DE DIAGNOSTIQUER L'ACCOUTUMANCE COMPORTEMENTALE ET PROCÉDÉ DE DÉTECTION DE RÉCEPTEUR DE SPHINGOSINE-1-PHOSPHATE POUR SON DIAGNOSTIC DE L'ACCOUTUMANCE COMPORTEMENTALE

(30) Priority: 17.01.2017 KR 20170008189
(43) Date of publication of application: 18.07.2018
(73) Proprietor: Korea Institute of Science and Technology, Seoul 02792 (KR)
(72) Inventor: LEE, Ji Eun, Seoul 02792 (KR); CHEON, Dong Huey, Seoul 02792 (KR); PARK, Yaeeun, Seoul 02792 (KR); PARK, Ji Soo, Seoul 02792 (KR); LEE, Cheolju, Seoul 02792 (KR); HAN, Ki-Cheol, Seoul 02792 (KR); KIM, Dai-Jin, Seoul 06591 (KR); CHUN, Jiwon, Seoul 06591 (KR); CHOI, Mi Ran, Seoul 06591 (KR); CHOI, Jung Seok, Seoul 06279 (KR)
(74) Representative: Handley, Matthew Edward

(56) References cited:
- TAKASHI AKIYAMA ET AL: "Immunohistochemical detection of sphingosine-1-phosphate receptor 1 in vascular and lymphatic endothelial cells", JOURNAL OF MOLECULAR HISTOLOGY, KLUWER ACADEMIC PUBLISHERS, DO, vol. 39, no. 5, 29 August 2008 (2008-08-29), pages 527-533, XP019642569, ISSN: 1567-2387, DOI: 10.1007/S10735-008-9193-Y
- Hirotake Nishimura ET AL: "Cellular Localization of Sphingosine-1-phosphate Receptor 1 Expression in the Human Central Nervous System", Journal of Histochemistry & Cytochemistry, 1 September 2010 (2010-09-01), pages 847-856, XP055405683, Los Angeles, CA DOI: 10.1369/jhc.2010.956409 Retrieved from the Internet: URL:https://www.ncbi.nlm.nih.gov/pmc/artic les/PMC2924800/pdf/847.pdf
- TALMONT FRANCK ET AL: "Evaluation of commercial antibodies against human sphingosine-1-phosphate receptor 1", NAUNYN-SCHMIEDEBERG'S ARCHIVES OF PHARMACOLOGY, vol. 387, no. 5, May 2014 (2014-05), pages 427-431, XP009195471,
- TRIFILIEFF PIERRE ET AL: "Blunted Dopamine Transmission in Addiction: Potential Mechanisms and Implications for Behavior", SEMINARS IN NUCLEAR MEDICINE, ELSEVIER, AMSTERDAM, NL, vol. 47, no. 1, 13 October 2016 (2016-10-13), pages 64-74, XP029848086, ISSN: 0001-2998, DOI: 10.1053/J.SEMNUCLMED.2016.09.003
- YAU YVONNE H C ET AL: "Gambling disorder and other behavioral addictions: recognition and treatment.", HARVARD REVIEW OF PSYCHIATRY, vol. 23, no. 2, March 2015 (2015-03), pages 134-146, XP009195470, ISSN: 1465-7309

## Description

### CROSS-REFERENCE TO RELATED APPLICATION

This application claims the benefit of Korean Patent Application No. 10-2017-0008189, filed on January 17, 2017, in the Korean Intellectual Property Office.

### BACKGROUND

### 1. Field

The present disclosure relates to a composition or a kit for diagnosing behavioral addictions, and a method of detecting sphingosine 1-phosphate receptor 1 for diagnosis of behavioral addictions using the same.

### 2. Description of the Related Art

Behavioral addictions are addictions to any behavior, process, or activity, such as gambling addiction, sex addiction, pathological use of Internet or game, etc. Brain circuits are involved in behavioral addictions, and resistance and withdrawal may occur. Impulsivity dominates at the early stages of development, and compulsivity is the key to maintain behavior. In modern society, Internet addiction, game addiction, and obsessive addiction, together with alcoholism and drug addiction, are becoming more prominent.

Biomarkers for psychiatric symptoms, for example, autism, Parkinson's disease, dementia, attention deficit hyperactivity disorder (ADHD), alcoholism, drug addiction, obsessive-compulsive disorder, etc., are known (US Patent Publication NO. 2005/0084880A1 (2005.04.21), Korean Patent NO. 10-1157526 (2016.06.27), Trifilieff et al., Seminars in Nuclear Medicine, vol. 47, no. 1, pages 64 - 74, etc.). However, for diagnosis of behavioral addiction, in particular, Internet addiction or game addiction, brain imaging through magnetic resonance imaging (MRI) or self-assessment through a questionnaire survey is mainly used, but there are no effective biomarkers for diagnosis thereof.

Accordingly, there is a need for the development of biomarkers capable of diagnosing behavioral addictions easily and inexpensively with high accuracy and specificity, not an expensive and inconvenient brain imaging diagnosis or a selfdiagnosis method based on subjective assessment.

### SUMMARY

The present invention is defined in the independent claims, to which reference should now be made. Advantageous embodiments are set out in the sub claims. Provided is a composition for diagnosing behavioral addictions.

Provided is a kit for diagnosing behavioral addictions.

Provided is a method of detecting sphingosine 1-phosphate receptor 1 for the diagnosis of behavioral addictions.

### BRIEF DESCRIPTION OF THE DRAWINGS

These and/or other aspects will become apparent and more readily appreciated from the following description of the embodiments, taken in conjunction with the accompanying drawings in which:
FIG. 1 is a graph showing results of high-performance liquid chromatography in which the high-abundance proteins were removed from a serum sample using immunoaffinity column (x axis: time (min), y axis: milli Absorbance Unit (mAU));
FIG. 2A is an SDS-PAGE gel image before and after depletion of the high-abundance proteins from four serum samples of normal control group, and FIG. 2B is an SDS-PAGE gel image before and after depletion of the high-abundance proteins from four serum samples of Internet or game addiction group (left: before depletion of the high-abundance proteins from four individual serum samples, right: after depletion of the high-abundance proteins from four individual serum samples, M: molecular weight marker (KDa), 1 to 4: Lanes 1 to 4);
FIG. 3A is an image obtained by performing an antibody array of 507 kinds of proteins, FIG. 3B is an image showing a comparison of antibody array spots of S1PR1 between the control group and the Internet or game addiction group, and FIG. 3C is a bar graph showing relative intensities of antibody array spots of S1PR1; and
FIG. 4A is a graph (p=0.0047) obtained by analyzing the quantity of S1PR1 in the Internet or game addiction group (addiction group) or in the normal control group (control group) by ELISA, and FIG. 4B is an ROC curve of S1PR1.

### DETAILED DESCRIPTION

An aspect provides a composition for diagnosing behavioral addictions, the composition including an agent capable of measuring an expression level of sphingosine 1-phosphate receptor 1 (S1PR1) protein or a fragment thereof.

The term "behavioral addiction" refers to an addiction to any behavior, process, or activity. Behavioral addictions may involve resistance and withdrawal. The behavioral addictions may be selected from the group consisting of Internet addiction, smart phone addiction, game addiction, gambling addiction, religious addiction, sex addiction, shopping addiction, and work addiction. Internet addiction refers to an addiction state which is caused by pathological or compulsive Internet use to affect physical, mental, or social life. Game addiction refers to an addiction state which is caused by pathological or compulsive game immersion to affect physical, mental, or social life. The Internet or game includes those by use of all mediums such as computers, mobile phones, smart phones, TVs, etc.

S1PR1 is a G-protein-coupled receptor binding to a cell signaling molecule sphingosine 1-phosphate (S1P). S1PR1 may be also called endothelial differentiation gene 1 (EDG1), CD363, CHEDG1, D1S3362, ECGF1, or EDG-1. S1PR1 may be a protein including seven transmembrane helices and three loops. S1PR1 is a protein encoded by human S1PR1 gene. S1PR1 may include an amino acid sequence of Uniprot ID P21453 in humans. S1PR1 may include an amino acid sequence of Uniprot ID 008530 in mice. Antibodies and primers for measuring the expression of S1PR1 are described, for example, in Takashi Akiyama et al., Journal Of Molecular Histology, vol. 39, no. 5, pages 527 - 533, and Hirotake Nishimura et al., Journal of Histochemistry & Cytochemistry, 1 Sept 2010, pages 847 - 856. Antibodies against human S1PR1 are also described in Talmont et al., NAUNYN-SCHMIEDEBERG'S ARCHIVES OF PHARMACOLOGY, vol. 387, no. 5, pages 427 - 431.

The fragment may be an immunogenic polypeptide as a part of S1PR1 protein.

The agent may be an antibody or an antigen-binding fragment thereof specifically binding to S1PR1 protein or a fragment thereof. The antibody may be a polyclonal antibody or a monoclonal antibody. The term "antibody" may be used interchangeably with "immunoglobulin". The antibody may be a polyclonal antibody or a monoclonal antibody. The antibody may be a full-length antibody. The antigen-binding fragment refers to a polypeptide including an antigen-binding site. The antigen-binding fragment may be a single-domain antibody, Fab, Fab', or scFv. The antibody or antigen-binding fragment may be attached to a solid support. The solid support may be, for example, a surface of a metal chip, plate or well. The antibody or antigen-binding fragment may be an anti-S1PR1 antibody or an antigen-binding fragment thereof.

The agent may be a nucleotide including a polynucleotide which is identical or complementary to a polynucleotide encoding S1PR1 protein or a fragment thereof. The nucleotide may be a primer or a probe. The primer or the probe may be labeled with a fluorescent material, a chemiluminescent material, a radioactive isotope, etc. at the terminus or internally.

Another aspect provides a kit for diagnosing behavioral addictions, the kit including an agent capable of measuring an expression level of S1PR1 protein or a fragment thereof.

The kit may further include a sample required for the diagnosis of behavioral addictions. For immunological detection of the solid support, antibody, or antigen-binding fragment, the kit may include a substrate, an appropriate buffer solution, a color development enzyme, a fluorescent-labeled secondary antibody, or a color development substrate. For nucleotide detection, the kit may include a polymerase, a buffer, a nucleotide, a coenzyme, a fluorescent material, or a combination thereof. The polymerase may be, for example, Taq polymerase.

Still another aspect provides a method of detecting S1PR1 for the diagnosis of behavioral addictions, the method including measuring an expression level of S1PR1 protein or a fragment thereof in a biological sample which is separated from a subject suspected of having a behavioral addiction; and comparing the measured expression level with a protein expression level of a normal control group.

The method includes measuring an expression level of S1PR1 protein or a fragment thereof in a biological sample which is separated from a subject suspected of having a behavioral addiction.

The subject may be a mammal, for example, a human, a cow, a horse, a pig, a dog, a sheep, a goat, or a cat. The subject may be a subject suffering from a behavioral addiction or a subject suspected of having a behavioral addiction.

The biological sample refers to a sample obtained from the subject. The biological sample may be, for example, blood, plasma, serum, bone marrow fluid, lymphatic fluid, saliva, lachrymal fluid, mucosal fluid, amniotic fluid, or a combination thereof.

The measuring may include incubating the biological sample with an antibody specifically binding to S1PR1 protein or a fragment thereof.

The measuring may be performed by electrophoresis, immunoblotting, enzyme-linked immunosorbent assay (ELISA), protein chips, immunoprecipitation, microarray, northern blotting, polymerase chain reaction (PCR), or a combination thereof. The electrophoresis may be SDS-PAGE, isoelectric focusing, two-dimensional electrophoresis, or a combination thereof. The PCR may be real-time PCR or reverse transcription PCR.

The method includes comparing the measured expression level with a protein expression level of a normal control group.

In the method, a quantity of the measured expression level of S1PR1 may be decreased, compared with an expression level of S1PR1 derived from a sample of a normal control group. The normal control group means a negative control group having no behavioral addiction. For example, when the protein level of the detected S1PR1 protein is less than that of the S1PR1 protein of the normal control group, the subject may be diagnosed as having or at high risk of having a behavioral addiction, for example, Internet addiction or game addiction. The subject diagnosed as having a behavioral addiction, for example, Internet addiction or game addiction may be subjected to psychosocial therapy or drug treatment. The drug may be, for example, naltrexone, disulfiram, methadone, chlordiazepoxide, acamprosate, bupropion, varenicline, buprenorphine, or a combination thereof.

According to a composition or a kit for diagnosing behavioral addictions and a method of detecting S1PR1 for the diagnosis of behavioral addictions using the same according to an aspect, behavioral addictions, for example, Internet addiction or game addiction, may be simply diagnosed with high accuracy and specificity.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects.

Hereinafter, the present invention will be described in more detail with reference to Examples. However, these Examples are for illustrative purposes only, and the scope of the present invention is not intended to be limited by these Examples.

### Example 1. Identification of differentially expressed proteins among serum proteins of Internet or game addiction group

### 1. Preparation of samples of Internet or game addiction groups

Serum samples of Internet or game addiction patients (n=4) were collected from Seoul St. Mary's Hospital, Catholic University of South Korea. Serum samples of normal persons (n=4) who were not diagnosed as Internet or game addiction were collected as a negative control.

To prevent protein degradation or denaturation of the serum samples, a complete Mini EDTA-free protease inhibitor cocktail (Roche) as a protein degradation inhibitor were added to the collected serum immediately after collecting the serum. One tablet of the inhibitor was dissolved in 200 µl of a phosphate buffered saline (PBS) to prepare a 50 X stock according to the manufacturer's protocol, and then added to each serum sample at a final concentration of 1.5 X. The inhibitor-added serum samples were quantified and stored at -80°C before use in experiments.

### 2. Depletion of high-abundance proteins in serum samples

To remove abundant proteins in the serum, such as albumin or immunoglobulin G, the collected serum samples were depleted.

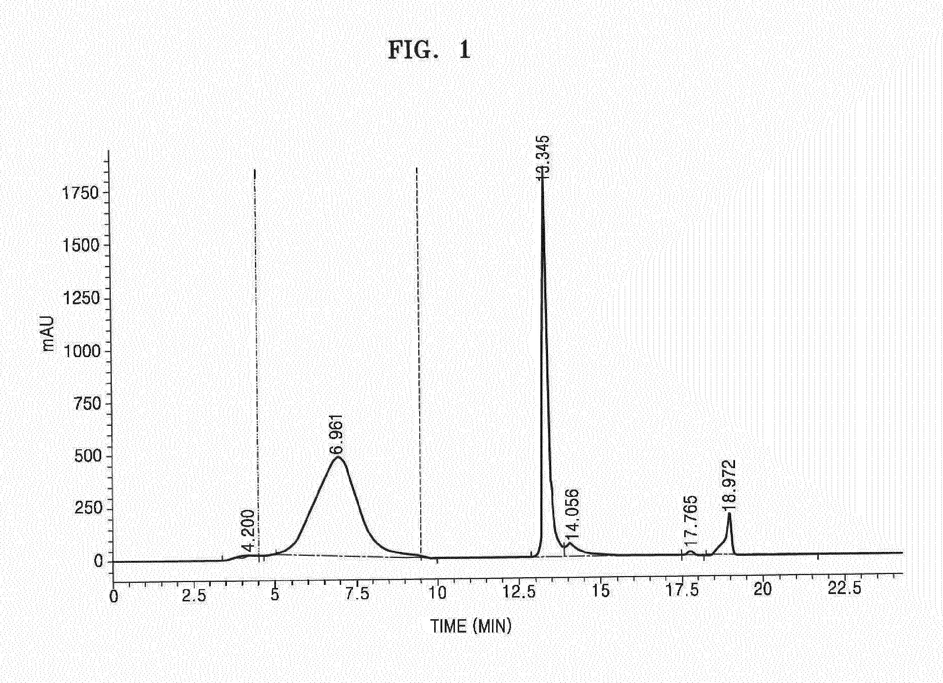

In detail, the serum samples collected in 1. were prepared at a concentration of 54.8 µg/µl which is a minimum concentration of a serum sample among 8 serum samples, respectively, and experiments were performed under the same conditions. The prepared serum samples were diluted 6 times with a buffer solution provided by the manufacturer, and each of the diluted samples was filtered using a 0.22 µm spin filter. 100 µl of the filtered sample was injected into a Multiple Affinity Removal System (MARS) 14 column (Agilent), and the top 14 high-abundance proteins (albumin, immunoglobulin G, alpha-1-antitripsin, immunoglobulin A, transferrin, haptoglobin, fibrinogen, alpha-2-macroglobulin, alpha-1-acid glycoprotein, immunoglobulin M, apolipoprotein A-I, apolipoprotein A-II, complement protein C3, and transthyretin) were removed from each sample was removed using an Agilent 1100 HPLC system. Results of measuring absorbance of the serum sample depleted of the top 14 high-abundance proteins by the MARS 14 column in real time are shown in FIG. 1 (-••-••-: a starting point for collecting the depleted serum, - - - -: an end point for collecting the depleted serum).

### 3. Concentration and quantification of depleted serum sample

A 3k centrifugal filter (Amicon Ultra, Millipore) was conditioned by adding 400 µl of water (HPLC grade, J.T Baker) twice and 400 µl of 10 mM HEPES buffer (pH 8.0) twice thereto.

Each of the serum samples depleted in 2. was diluted 5 times with 10 mM HEPES buffer. Each diluted sample was applied to the conditioned filter, and centrifuged at a speed of 14000x g and 10°C to concentrate the sample. Thereafter, the sample-containing filter was washed with 10 mM HEPES buffer 5 times. The centrifugal filter containing the depleted serum sample was turned over and put in a new tube, and centrifuged at a speed of 3000x g and 10°C for 5 minutes to recover the concentrated sample.

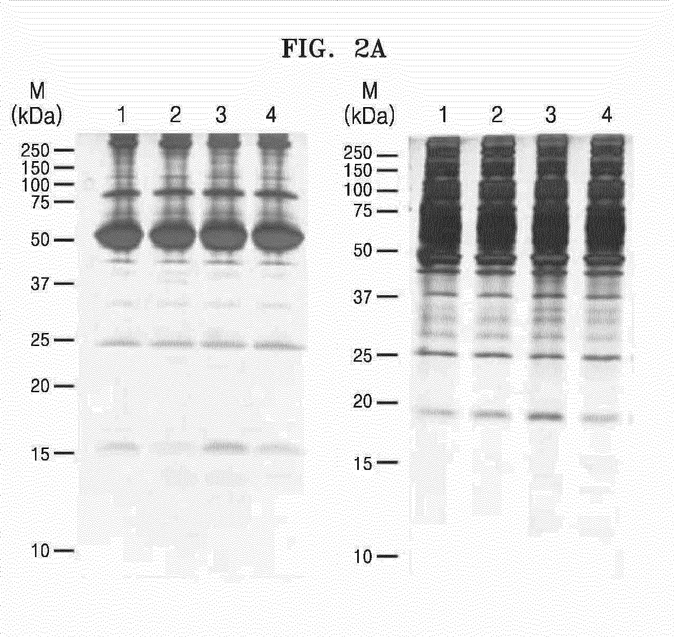

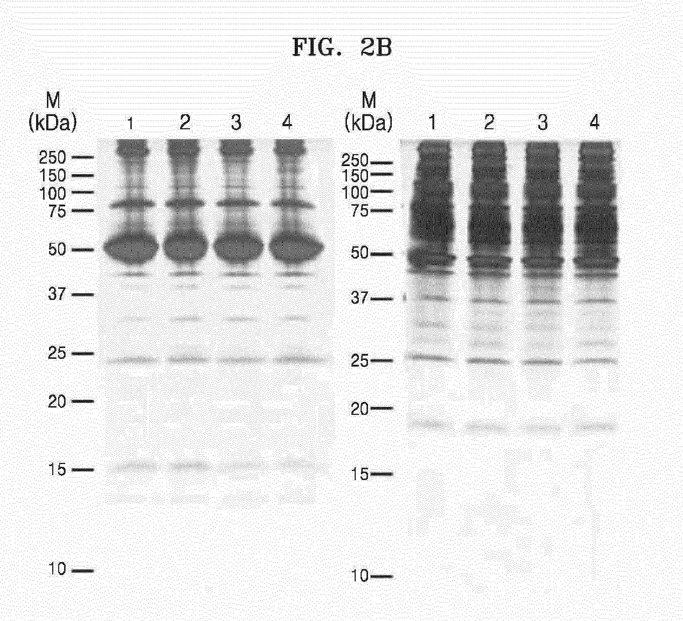

The concentrated samples were quantified by a bicinchoninic acid (BCA) assay. Equal amounts of the proteins were separated by SDS-PAGE, and the electrophoresed gels were stained by silver staining for visualization of each depleted serum sample. Images of the stained gels are shown in FIGS. 2A and 2B (FIG. 2A: SDS-PAGE gel image before and after depletion of the serum samples of the normal control group, FIG. 2B: SDS-PAGE gel image before and after depletion of the serum samples of the Internet or game addiction group).

### 4. Comparative analysis of protein expression from normal and internet or game addiction samples using antibody array

4 serum samples of the normal control group and 4 serum samples of the addiction group including Internet or game addicts were, respectively, pooled at a protein concentration of 500 µg/1.5 ml .

3.6 µl of a biotinylation reagent (RayBiotech, Inc.) was added to each of the pooled serum samples, and incubated for 30 minutes at room temperature, and then 5 µl of a stop solution (RayBiotech, Inc.) was added to terminate the reaction. The reactant was filtered through a size exclusion column (RayBiotech, Inc.) to remove remaining biotin in the sample.

The human antibody 507 arrays (membrane type, available from RayBiotech, Inc.) were blocked for 1 hour at room temperature by a blocking buffer solution. The blocking buffer solution was also added to 1.5 ml of a biotin-labeled sample to be diluted at a total volume of 14 ml. Each sample from either control or addiction group was applied to the array, and incubated at 4°C overnight.

Thereafter, the array was washed with a washing buffer included in the antibody array kit, and a streptavidin-HRP conjugated solution (RayBiotech, Inc.) was added to the array, followed by incubation at room temperature for 2 hours. The array was washed, and signals from the array were detected using chemiluminescence.

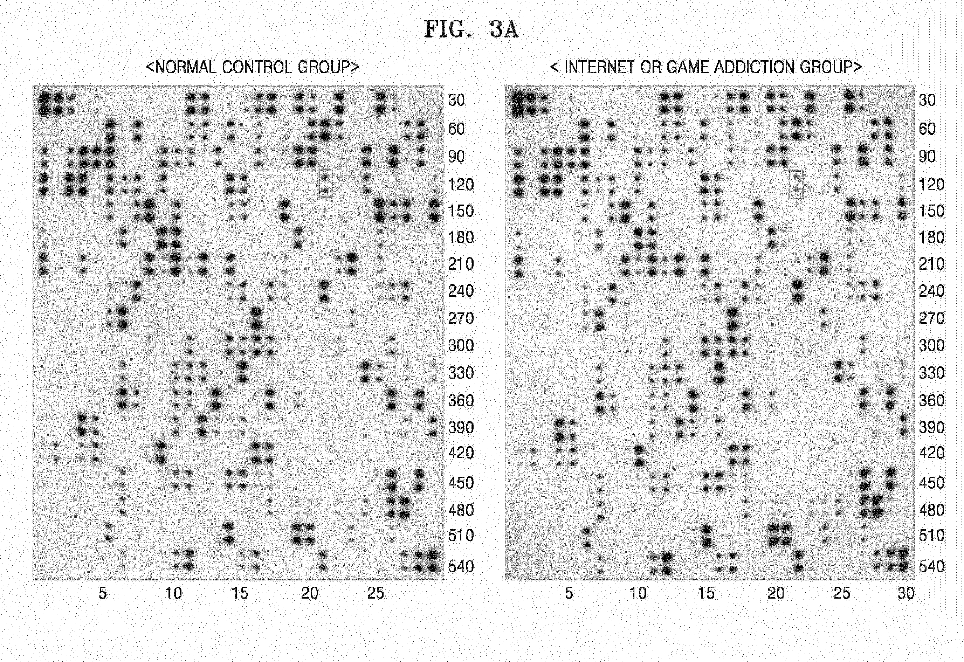

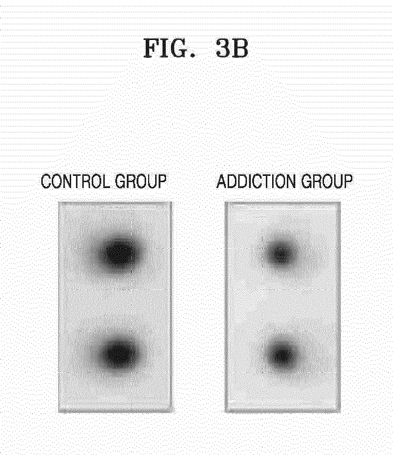

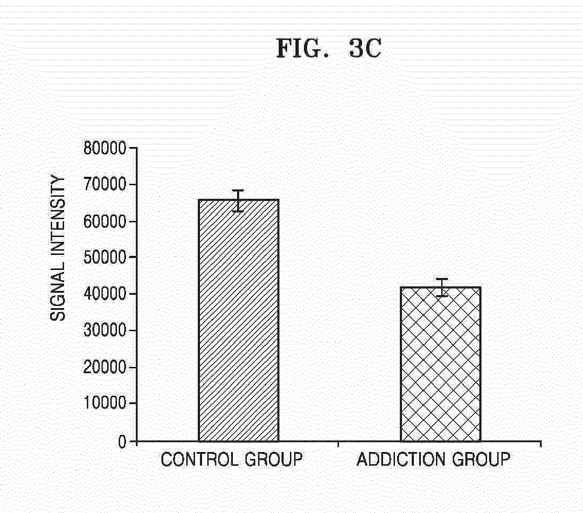

Results of the antibody arrays containing 507 proteins from the control and the Internet or game addiction samples are shown in FIG. 3A. As shown in FIG. 3A, proteins, which were differentially expressed in the samples of the normal control group and the Internet or game addiction group, were observed. Among the proteins, which were differentially expressed in the Internet or game addiction group, S1PR1 was selected as a biomarker, and an antibody array image of the S1PR1 protein is shown in FIG. 3B. In the antibody array image of the S1PR1 protein, relative intensities of the spots were quantified by using TotalLab 1D analysis software (Nonlinear Dynamics), and relative intensities of the spots in the control group and the Internet or game addiction group are shown in FIG. 3C.

As shown in FIG. 3C, the S1PR1 expression was significantly decreased in the Internet or game addiction group, compared to the control group.

### 5. Enzyme-linked immunosorbent assay for differential expression

An expression pattern of the S1PR1 protein selected as a biomarker candidate in 4. was assayed. For assay, samples of 39 persons of the control group and 15 persons of the Internet or game addiction group provided by Seoul St. Mary's Hospital, Catholic University of South Korea were used to perform experiments.

Serum samples of the control group and the Internet or game addiction group, which were diluted with PBS at a ratio of 1:20, and each 100 µl of S1PR1 standard (Wuhan USCN Business Co., Ltd) at a concentration of 10 ng/ml, 5 ng/ml, 2.5 ng/ml, 1.25 ng/ml, 0.625 ng/ml, 0.312 ng/ml, 0.156 ng/ml, or 0 ng/ml were prepared, and loaded in a 96-well ELISA plate (Wuhan USCN Business Co., Ltd). The ELISA plate was incubated at 37°C for 1 hour. The sample and standards remaining after reaction were removed, and then 100 µl of a detection reagent A (Wuhan USCN Business Co., Ltd) diluted at a ratio of 1:100 was added to the ELISA plate, followed by incubation at 37°C for 1 hour. The sample and standards remaining after reaction were removed, and then 300 µl of washing buffer was added to each well, which was washed three times. Thereafter, 100 µl of a detection reagent B (Wuhan USCN Business Co., Ltd) diluted at a ratio of 1:100 was added to each well, followed by incubation at 37°C for 30 minutes. The sample and standards remaining after reaction were removed, and the wells were washed with a washing buffer 5 times. 90 µl of a substrate solution (Wuhan USCN Business Co., Ltd) was added to each well, and the ELISA plate was incubated at 37°C for 10 -20 minutes in the dark. When the color of the sample and standards were changed to blue, 50 µl of a stop solution (Wuhan USCN Business Co., Ltd) was added to the ELISA plate. Absorbance at 450 nm was measured.

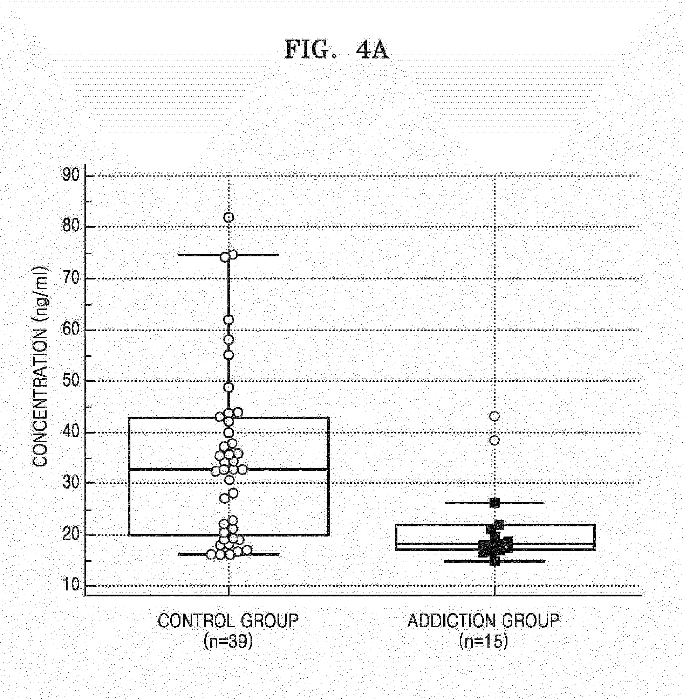

From the measured value, a concentration of the sample was calculated, and analyzed by a Mann-Whitney U-test. Results are shown in FIG. 4A. As shown in FIG. 4A, the S1PR1 expression was significantly decreased in the Internet or game addiction group, compared to the normal control group.

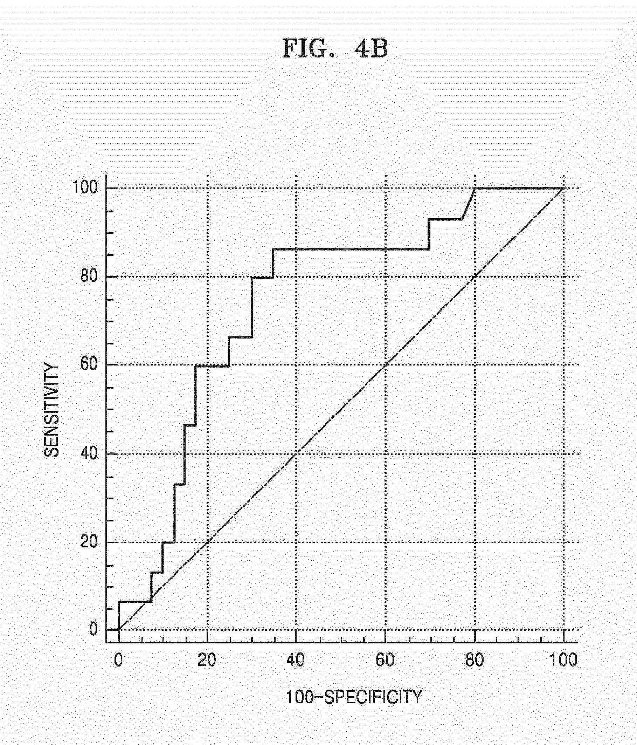

Further, a receiver operating characteristic (ROC) curve of S1PR1 is shown in FIG. 4B. In the ROC curve, an area under curve (AUC) value was 0.749 and a p value was 0.001, indicating that S1PR1 has excellent accuracy as a biomarker for Internet or game addiction.

It should be understood that embodiments described herein should be considered in a descriptive sense only and not for purposes of limitation.

## Claims

1. Use of a composition for the diagnosis of behavioral addictions, the composition comprising an agent capable of measuring an expression level of sphingosine 1-phosphate receptor 1 (S1PR1) protein, in a biological sample,
wherein the behavioral addiction is selected from the group consisting of Internet addiction and game addiction, and
wherein the biological sample is blood, plasma, serum, or a combination thereof.

2. The use as claimed in claim 1, wherein the agent is:
an antibody or an antigen-binding fragment thereof specifically binding to S1PR1 protein; or
a nucleotide comprising a polynucleotide which is identical or complementary to a polynucleotide encoding S1PR1 protein.

3. The use as claimed in claim 2, wherein the antibody is a polyclonal antibody or a monoclonal antibody.

4. The use as claimed in claim 2, wherein the nucleotide is a primer or a probe.

5. A method for diagnosis of behavioral addictions by detecting S1PR1, the method comprising:
measuring an expression level of S1PR1 protein in a biological sample which is separated from a subject suspected of having a behavioral addiction; and
comparing the measured expression level with a S1PR1 protein expression level of a normal control group,
wherein the behavioral addiction is selected from the group consisting of Internet addiction and game addiction,
wherein the biological sample is blood, plasma, serum, or a combination thereof.

6. The method of claim 5, wherein the measuring comprises incubating the biological sample with an antibody specifically binding to S1PR1 protein.

7. The method of claim 5, wherein the measuring is performed by electrophoresis, immunoblotting, enzyme-linked immunosorbent assay (ELISA), protein chips, immunoprecipitation, microarray, northern blotting, polymerase chain reaction (PCR), or a combination thereof.

## Patentansprüche

1. Verwendung einer Zusammensetzung zur Diagnose von Verhaltensabhängigkeiten, wobei die Zusammensetzung ein Mittel umfasst, das einen Expressionswert des Proteins Sphingosin-1-phosphat-Rezeptor 1 (S1PR1) in einer biologischen Probe messen kann,
wobei die Verhaltensabhängigkeit ausgewählt ist aus der Gruppe bestehend aus Internetsucht und Spielsucht, und
wobei die biologische Probe Blut, Plasma, Serum oder eine Kombination davon ist.

2. Verwendung nach Anspruch 1, wobei das Mittel folgendes ist:
ein Antikörper oder ein Antigenbindungsfragment davon, das spezifisch an das Protein S1PR1 bindet; oder
ein Nukleotid, das ein Polynukleotid umfasst, das identisch oder komplementär ist zu einem für das Protein S1PR1 codierenden Polynukleotid.

3. Verwendung nach Anspruch 2, wobei der Antikörper ein polyklonaler Antikörper oder ein monoklonaler Antikörper ist.

4. Verwendung nach Anspruch 2, wobei das Nukleotid ein Primer oder eine Sonde ist.

5. Verfahren zur Diagnose von Verhaltensabhängigkeiten durch den Nachweis von S1PR1, wobei das Verfahren folgendes umfasst:
Messen eines Expressionswertes des Proteins S1PR1 in einer biologischen Probe, die von einem Subjekt getrennt wird, bei dem eine Verhaltensabhängigkeit vermutet wird; und
Vergleichen des gemessenen Expressionswertes mit einem Expressionswert des Proteins S1PR1 einer normalen Kontrollgruppe,
wobei die Verhaltensabhängigkeit ausgewählt ist aus der Gruppe bestehend aus Internetsucht und Spielsucht, und
wobei die biologische Probe Blut, Plasma, Serum oder eine Kombination davon ist.

6. Verfahren nach Anspruch 5, wobei das Messen das Inkubieren der biologischen Probe mit einem Antikörper umfasst, der spezifisch an das Protein S1PR1 bindet.

7. Verfahren nach Anspruch 5, wobei das Messen durchgeführt wird durch Elektrophorese, Immunoblotting, Enzyme-Iinked Immunosorbent Assay (ELISA), Protein-Chips, Immunpräzipitation, Mikroarray, Northern Blotting, Polymerasekettenreaktion (PCR) oder eine Kombination davon.

## Revendications

1. Utilisation d'une composition pour le diagnostic d'accoutumances comportementales, la composition comprenant un agent capable de mesurer un niveau d'expression de la protéine du récepteur 1 de sphingosine-1-phosphate (S1PR1), dans un échantillon biologique,
l'accoutumance comportementale étant sélectionnée dans le groupe constitué par l'accoutumance à Internet et l'accoutumance aux jeux, et
l'échantillon biologique étant du sang, du plasma, du sérum, ou une combinaison de ceux-ci.

2. Utilisation selon la revendication 1, l'agent étant :
un anticorps ou un fragment de liaison à l'antigène de celui-ci se liant spécifiquement à la protéine S1PR1 ; ou
un nucléotide comprenant un polynucléotide qui est identique à ou complémentaire d'un polynucléotide codant la protéine S1PR1.

3. Utilisation selon la revendication 2, l'anticorps étant un anticorps polyclonal ou un anticorps monoclonal.

4. Utilisation selon la revendication 2, le nucléotide étant une amorce ou une sonde.

5. Procédé de diagnostic d'accoutumances comportementales par détection de S1PR1, le procédé comprenant les étapes consistant à :
mesurer un niveau d'expression de la protéine S1PR1 dans un échantillon biologique qui est séparé d'un sujet soupçonné d'avoir une accoutumance comportementale ; et
comparer le niveau d'expression mesuré avec un niveau d'expression de la protéine S1PR1 d'un groupe de contrôle normal,
l'accoutumance comportementale étant sélectionnée dans le groupe constitué par l'accoutumance à Internet et l'accoutumance aux jeux, et
l'échantillon biologique étant du sang, du plasma, du sérum, ou une combinaison de ceux-ci.

6. Procédé selon la revendication 5, l'étape consistant à mesurer comprenant l'étape consistant à incuber l'échantillon biologique avec un anticorps se liant spécifiquement à la protéine S1PR1.

7. Procédé selon la revendication 5, l'étape consistant à mesurer étant réalisée par électrophorèse, buvardage, essai d'immuno-absorption enzymatique (ELISA), puces à protéines, immunoprécipitation, microréseau, transfert de Northern, réaction en chaîne de la polymérase (PCR), ou une combinaison de ceux-ci.
